# EUROPEAN PATENT APPLICATION

(11) **EP 1 024 364 A1**
(43) Date of publication of application: **02.08.2000**
(21) Application number: 99101377.2
(22) Date of filing: 26.01.1999
(51) Int. Cl.: G01N 33/68

(54) **Methods of diagnosing or prognosing Alzheimer's disease**

(71) Applicant: Nitsch, Roger M., Prof. Dr., 20146 Hamburg (DE)
(72) Inventor: Nitsch, Roger M., Prof. Dr, 20146 Hamburg (DE); Growdon, John H., Dr., Chestnuthill, MA 02167 (US)
(74) Representative: Meyers, Hans-Wilhelm, Dr.Dipl.-Chem.

(57) **Abstract**

A method for diagnosing or prognosing Alzheimer's disease in a subject, or determining whether a subject is at increased risk of developing Alzheimer's disease, comprising:
determining a level, or an activity, or both said level and said activity, of at least one substance which is selected from the group consisting of cystatin C, a variant thereof, a transcription product of a cystatin C gene, and a variant thereof, in a sample from said subject; and
comparing said level, or said activity, or both said level and said activity, of at least one of said substances to a reference value representing a known disease or health status,
thereby diagnosing or prognosing said Alzheimer's disease in said subject, or determining whether said subject is at increased risk of developing Alzheimer's disease.

## Description

Alzheimer's disease (AD), first described by the Bavarian psychiatrist Alois Alzheimer in 1907, is a progressive neurological disorder which begins with short term memory loss and proceeds to loss of cognitive functions, disorientation, impairment of judgment and reasoning and, ultimately, dementia. It is the most common cause of dementia. The neuropathology is characterized by the formation in brain of amyloid plaques and neurofibrillary tangles. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2,* as well as to mutations of the amyloid precursor gene *APP.* The majority of AD patients have no obvious family history and are classified as sporadic AD.

In the search for biochemical changes in patients with neurological disorders such as AD, analysis of cerebrospinal fluid (CSF) may be a useful method, since the CSF is continuous with the extracellular fluid of the brain. Therefore, a plurality of studies aiming at the analysis of central nervous system (CNS) specific proteins in CSF were performed in order to find biochemical markers for neuronal and synaptic function and pathology in degenerative brain disorders. Further studies were directed to the identification of possible genetic risk factors including polymorphisms in the human cystatin C gene. The human cystatin C gene *(CST3)* maps to chromosome 20p11.2, it contains three exons, and a Sst II polymorphic site within the 5' flanking sequence that is in linkage disequilibrium with a second *Sst* II polymorphism within exon 1 of the gene (Balbin and Abrahamson, Hum. Genet. 81, 751 - 752, 1991; Balbin et al., Hum. Genet. 92, 206 - 207, 1993; Abrahamson et al., FEBS Lett. 216, 229 - 233, 1987). The open reading frame of *CST3* encodes a 120-residue protein with a molecular mass of 13.3 kDa and a pl of 8.75 (Abrahamson et al., J. Biol. Chem. 261, 11282 - 11289, 1986; Abrahamson et al., FEBS Lett. 216, 229 - 233, 1987). The mature molecule contains intramolecular disulfide bonds, it is partially hydroxylated, no other common post-translational modifications were observed (Grubb and Lofberg, Proc. Natl. Acad. Sci., USA, 79, 3024 - 3027, 1982; Asgeirsson et al., Biochem. J., 329, 497 - 503, 1998). Cystatin C is distributed extensively in the body fluids and is suspected of playing a role in extracellular functions, such as the modulation of inflammatory reactions. It is known to exist in cell types, such as astrocytes, macrophages, and choroid plexus cells. Cystatin C also is a quantitatively dominating cysteine protease inhibitor of CSF whose concentration is five times higher than that of plasma. It binds to and regulates proteolytic activities of cathepsins which have been associated with brain amyloid plaques in Alzheimer's disease, and implicated in the proteolytic processing of the amyloid precursor protein. It has been described that human cystatin C undergoes dimerization before unfolding. Dimerization leads to a complete loss of its activity as a cysteine proteinase inhibitor (Ekiel et al., J. Mol. Biol. 271, 266 - 277, 1997). In addition to the termination of biological activity, dimerization of cystatin C is associated with brain amyloid formation in the Islandic form of hereditary cerebral hemorrhage with amyloidosis caused by an inherited mutation (L68Q) within the coding region of *CST3.*

The production rate of cystatin C is remarkably constant and its plasma concentration can therefore be used as a reliable measure of the glomerular filtration rate (Grubb, Clinical Nephrology, Vol. 38, Suppl. No.1, 20 - 27, 1992).

Nagai et al. (Molecular and Chemical Neuropathology, Vol. 33, p. 63 - 78, 1998) found out that cerebral amyloid angiopathy (CAA) patients, on whose cerebral blood vessels cystatin C colocalized with β-protein, had low concentration of cystatin C in their cerebrospinal fluid (CSF). DNA analysis of these patients showed no point mutations in the cystatin C gene in contrast to hereditary cerebral hemorrhage with amyloidosis in Iceland (HCHWA-I) characterized by a variant cystatin C with an amino acid substitution at position 68 and a corresponding point mutation at the genetic level. An abnormally low level of cystatin C in CSF as a diagnostic marker for the hereditary form of brain hemorrhage associated with amyloidosis in Iceland was confirmed by Shimode and co-workers who developed an assay for cystatin C to be used in the diagnosis of patients with cerebral amyloid angiopathy and brain hemorrhage (Stroke, Vol. 22, 860 - 866, 1991).

The use of determinations of the CSF concentration of cystatin C - also termed γ-trace - in the diagnosis of hereditary cerebral hemorrhage with γ-trace-amyloidosis was described by Grubb and Lofberg (Scand. J. Clin. Lab. Invest., 45, Suppl. 177: 7 - 13, 1985).

Shimode et al. (Stroke, Vol. 27, 1417 - 1419, 1996) further found out that measurement of cystatin C in CSF by enzyme-linked immunosorbent assay (ELISA) is a useful method of diagnosing leukoencephalopathy related to sporadic cystatin-C type cerebral amyloid angiopathy.

Regarding HCHWA-I, identification of the disease-causing mutation and specific diagnosis by polymerase chain reaction based analysis has been described by Abrahamson et al. (Hum. Genet. 89: 377 - 380, 1992).

Davidsson et al. (J. Neural. Transm., 104: 711 - 720, 1997) describe a procedure for detecting brain-specific proteins in cerebrospinal fluid. After three affinity chromatography steps, intended to remove interfering serum proteins from CSF, micro-reversed HPLC revealed four major peaks, which by both Western blotting and mass spectrometric analyses were found to correspond to β2-micro-globulin, cystatin C, transthyretin (TTR) and asialotransferrin. When comparing these peaks in CSF from Alzheimer's disease patients and age-matched healthy controls, only a reduction of the brain-specific pl 5.7 form of TTR and an increase in the pl 5.4 form of TTR was found.

Lofberg et al. (J. Neurol. 223, 159- 170, 1980) disclose the existence of a connection between the cerebrospinal fluid (CSF) and plasma level of γ-trace and the age of an individual. These results emphasize the necessity of using age-matched reference values when CSF and plasma levels of γ-trace are to be evaluated in different groups of patients. Children and adults with well defined neurological disorders were also examined in regard to their levels of γ-trace. These children suffered from acute aseptic meningitis; or meningococcal meningitis; or group B streptococcal septicemia and meningitis; or meningeal lymphoma; or infantile myoclonic seizures; or later developed cerebral palsy syndromes. The adults suffered from multiple sclerosis; or cerebral infarctions; or transitory ischemic attack; or reversible ischemic neurological deficit; or intracerebral, subdural or subarachnoid hemorrhage; or cerebral atrophy; or acoustic neuroma; or intramedullary gliomas; or intraspinal neuroma, or meningeal carcinomatosis; or Hodgkin's disease; or meningitis; or encephalitis; or chorioretinitis; or myelitis; or systemic lupus erythematosus; or arteritis with cerebral symptoms; or Guillain-Barré syndrome; or epilepsy; or polyneuropathy; or intervertebral disc protusion. Significantly increased CSF levels of γ-trace and increased plasma concentration of γ-trace were found in infectious disorders, i.e. meningitis, encephalitis, chorioretinitis and myelitis. Increased γ-trace concentration in plasma were seen in the above mentioned cerebrovascular disorders, i.e. cerebral infarction, transitory ischemic attack, reversible ischemic neurological deficit, intracerebral, subdural or subarachnoid hemorrhage, as well as in infectious disorders. Lofberg did not examine any patients suffering from Alzheimer's disease.

As Alzheimer's disease is a growing social and medical problem, there is a strong need for methods of diagnosing or prognosing said disease in subjects as well as for methods of treatment. In addition, there is a strong need for identifying inherited risk factors that increase the susceptibility of getting AD (susceptibility gene).

In one aspect, the invention features a method for diagnosing or prognosing Alzheimer's disease in a subject, or determining whether a subject is at increased risk of becoming diseased with Alzheimer's disease. The method includes: determining a level, or an activity, or both said level and said activity, of at least one substance which is selected from the group consisting of cystatin C, a variant thereof, a transcription product of a cystatin C gene, and a variant thereof, in a sample from said subject; and comparing said level, or said activity, or both said level and said activity, of at least one of said substances to a reference value representing a known disease or health status, thereby diagnosing or prognosing said Alzheimer's disease in said subject, or determining whether said subject is at increased risk of becoming diseased with Alzheimer's disease.

It might be preferred that said subject has previously been determined to have one or more factors indicating that such subject is afflicted with Alzheimer's disease.

In preferred embodiments, the body fluid is e.g. cerebrospinal fluid, saliva, urine or serum plasma. It is particularly preferred to use cerebrospinal fluid. According to the present invention, an increase of the level of cystatin C in said cerebrospinal fluid from the subject relative to a reference value indicates a diagnosis, or prognosis, or increased risk of said Alzheimer's disease in said subject. As shown in Example 1, CSF levels of cystatin C, and in particular in its monomeric form (which is the one with biological activity), are significantly elevated in AD as compared to normal control subjects or to patients with non-AD neurological diseases, indicating a role of cystatin C for the early diagnosis of AD. Because CSF levels of cystatin C measured by both ELISA and Western blotting were significantly lower in both non-AD neurodegenerative and infectious brain diseases, compared to AD, non-specific effects of neurodegeneration or inflammation on CSF levels of cystatin C are highly unlikely (see Example 1).

In preferred embodiments, measurement of the level of transcription products of the cystatin C gene, or a variant thereof, is performed in body cells using Northern blots with probes specific for the cystatin C gene or said variant. Quantitative PCR with primer combinations to amplify cystatin C gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, said level or activity of cystatin C, or a variant thereof, is detected using an immunoassay. These assays can e.g. measure the amount of binding between cystatin C and an anti-cystatin C antibody by the use of enzymatic, chromodynamic, radioactive, or luminescent labels which are attached to either the anti-cystatin C antibody or a secondary antibody which binds the anti-cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect cystatin C or a variant thereof. It is preferred that it does not substantially interact with any other protein present in said sample. It is particularly preferred to include specific antibodies or ligands which differentiate monomers from dimers or oligomers of cystatin C. As is it shown in Example 1, the detection of the monomeric form of cystatin C may be more specific to AD than measuring dimers or oligomers. Measures might be significantly improved with monomer-specific ELISAs.

Monoclonal antibodies capable of recognizing cystatin C or a variant thereof can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; ; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are directed to an epitope of cystatin C or a variant thereof, such that the complex formed between the antibody and cystatin C, or between the antibody and said variant, can be recognized in detection assays. The term "antibodies" encompasses all forms of antibodies known in the art, such as polyclonal , monoclonal, chimeric, recombinatorial, single chain antibodies as well as fragments thereof which specifically bind to cystatin C, or to a variant thereof. It is particularly preferred to use specific antibodies that selectively detect cystatin C monomers, dimers or oligomers, respectively. High-affinity ligands can be prepared by using derivatives of cathepsins which are the natural substrates of cystatin C biological activity.

It is further preferred to determine e.g. the level of cystatin C and its activity on basis of an enzymatic assay. As described above, cystatin C is a cysteine protease inhibitor which binds to and regulates proteolytic activities of cathepsins. A suitable enzymatic assay can therefore be built upon the enzymatic activity of cathepsins indicating the absense or presence of different levels of cystatin C in its active, monomeric form. It is preferred to use amyloid precursor protein (APP) as a substrate. The generation of A-beta peptides as educts can be measured.

The determination of the level or activity of cystatin C, or a variant thereof, can also be performed on basis of a binding assay. Suitable binding partners include cathepsins or fragments thereof, peptides, peptidomimetics, antibodies and other chemical probes which can specifically recognize cystatin C. It is in general particularly preferred to determine cystatin C in its monomeric form.

If luminescent labels are used in any detection assay, it is preferred to use a confocal optical set-up.

In preferred embodiments, the reference value can be that of a level, or an activity, or both said level and said activity, of at least one substance which is selected from the group consisting of cystatin C, a variant thereof, a transcription product of a cystatin C gene, and a variant thereof, in a sample, preferably a body fluid, from a subject not suffering from Alzheimer's disease. The healthy subject can be of the same weight, age, and gender as the subject who is being diagnosed or prognosed for Alzheimer's disease, or for whom an increased risk of becoming diseased with Alzheimer's disease is determined. In some cases, it might be preferred to use a reference value from the subject which is diagnosed.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In another aspect, the invention features, a method of monitoring the progression of Alzheimer's disease in a subject. The method includes: determining a level, or an activity, or both said level and said activity, of at least one substance which is selected from the group consisting of cystatin C, a variant thereof, a transcription product of a cystatin C gene, and a variant thereof in a sample, preferably a body fluid, from the subject; and comparing said level, or said activity, or both said level and said activity, of at least one of said substances to a reference value representing a known disease or health status, thereby monitoring the progression of said Alzheimer's disease in said subject. In a preferred embodiment, the level, or the activity, or both said level and said actitivity, of at least one of said substances in a sample is determined at least twice, e.g. at two points which are weeks or months apart. The levels or activities at these two time points are compared in order to monitor the progression of Alzheimer's disease. It might further be preferred to compare a level, or an activity, or both said level and said activity of at least one substance which is selected from the group consisting of cystatin C, a variant thereof, a transcription product of a cystatin C gene, and a variant thereof, in said sample with a level, an activity, or both said level and said activity, of at least one of said substances in a series of samples taken from said subject over a period of time. In further preferred embodiments, said subject receives a treatment prior to one or more of said sample gatherings.

It might be preferred that said subject has previously been determined to have one or more factors indicating that such subject is afflicted with Alzheimer's disease.

In preferred embodiments, the body fluid is e.g. cerebrospinal, saliva, urine or serum plasma. It is particularly preferred to use cerebrospinal fluid. According to the present invention, an increase of the level of cystatin C in said cerebrospinal fluid from the subject relative to a reference value indicates a diagnosis, or prognosis, or increased risk of said Alzheimer's disease in said subject. As shown in Example 1, CSF levels of cystatin C, and in particular in its monomeric form (which is the one with biological activity), are significantly elevated in AD as compared to normal control subjects or to patients with non-AD neurological diseases, indicating a role of cystatin C for the early diagnosis of AD. Because CSF levels of cystatin C measured by both ELISA and Western blotting were significantly lower in both non-AD neurodegenerative and infectious brain diseases, compared to AD, non-specific effects of neurodegeneration or inflammation on CSF levels of cystatin C are highly unlikely (see Example 1).

In preferred embodiments, measurement of the level of transcription products of the cystatin C gene, or a variant thereof, is performed in body cells using Northern blots with probes specific for the cystatin C gene or said variant. Quantitative PCR with primer combinations to amplify cystatin C gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, said level or activity of cystatin C, or a variant thereof, is detected using an immunoassay. These assays can measure e.g. the amount of binding between cystatin C and an anti-cystatin C antibody by the use of enzymatic, chromodynamic, radioactive, or luminescent labels which are attached to either the anti-cystatin C antibody or a secondary antibody which binds the anti-cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect cystatin C or a variant thereof. It is preferred that it does not substantially interact with any other protein present in said sample. It is particularly preferred to include specific antibodies or ligands which differentiate monomers from dimers or oligomers of cystatin C. As is it shown in Example 1, the detection of the monomeric form of cystatin C may be more specific to AD than measuring dimers or oligomers. Measures might be significantly improved with monomer-specific ELISAs.

Monoclonal antibodies capable of recognizing cystatin C, or a variant thereof, can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2nd ed., W. H. Freeman and Company, 1992; the contents of which are incorporated herein by reference). Monoclonal antibodies useful in the methods of the invention are directed to an epitope of cystatin C or a variant thereof, such that the complex formed between the antibody and cystatin C, or between the antibody and said variant, can be recognized in detection assays. The term "antibodies" encompasses all forms of antibodies known in the art, such as polyclonal , monoclonal, chimeric, recombinatorial, single chain antibodies as well as fragments thereof which specifically bind to cystatin C, or to a variant thereof. It is particularly preferred to use specific antibodies that selectively detect cystatin C monomers, dimers or oligomers, respectively. High-affinity ligands can be prepared by using derivatives of cathepsins which are the natural substrates of cystatin C biological activity.

It is further preferred to determine e.g. the level of cystatin C and its activity on basis of an enzymatic assay. As described above, cystatin C is a cysteine protease inhibitor which binds to and regulates proteolytic activities of cathepsins. A suitable enzymatic assay can therefore be built upon the enzymatic activity of cathepsins indicating the absense or presence of different levels of cystatin C in its active, monomeric form. It is preferred to use amyloid precursor protein (APP) as a substrate. The generation of A-beta peptides as educts can be measured.

The determination of the level or acitivity of cystatin C or a variant thereof can also be performed on basis of a binding assay. Suitable binding partners include cathepsins or fragments thereof, peptides, peptidomimetics, antibodies and other chemical probes which can specifically recognize cystatin C or said variant. It is in general particularly preferred to determine cystatin C in its monomeric form.

If luminescent labels are used in any detection assay, it is preferred to use a confocal optical set-up.

In preferred embodiments, the reference value can be that of a level, or an activity, or both said level and said activity, of at least one substance which is selected from the group consisting of cystatin C, a variant thereof, a transcription product of a cystatin C gene, and a variant thereof, in a sample, preferably a body fluid, from a subject not suffering from Alzheimer's disease. The healthy subject can be of the same weight, age, and gender as the subject who is being diagnosed or prognosed for Alzheimer's disease, or for whom an increased risk of becoming diseased with Alzheimer's disease is determined. In some cases, it might be preferred to use a reference value from the subject which is monitored.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In another aspect, the invention features, a method of evaluating a treatment for Alzheimer's disease. The method includes: determining a level, or an activity, or both said level and said activity, of at least one substance which is selected from a group consisting of cystatin C, a variant thereof, a transcription product of a cystatin C gene, and a variant thereof, in a sample obtained from said subject being treated for said Alzheimer's disease; and comparing said level, or said activity, or both said level and said activity, of at least one of said substances to a reference value representing a known disease or health status, thereby evaluating said treatment for said Alzheimer's disease. It is further preferred that a level, or an activity, or both said level and said activity of at least one of said substances is determined, before and after said treatment is administered to said subject.

It might be preferred that said subject has previously been determined to have one or more factors indicating that such subject is afflicted with Alzheimer's disease.

In preferred embodiments, the body fluid is e.g. cerebrospinal fluid, saliva, urine or serum plasma. It is particularly preferred to use cerebrospinal fluid. According to the present invention, an increase of the level of cystatin C in said cerebrospinal fluid from the subject relative to a reference value indicates a diagnosis, or prognosis, or increased risk of said Alzheimer's disease in said subject. As shown in Example 1, CSF levels of cystatin C, and in particular in its monomeric form (which is the one with biological activity), are significantly elevated in AD as compared to normal control subjects or to patients with non-AD neurological diseases, indicating a role of cystatin C for the early diagnosis of AD. Because CSF levels of cystatin C measured by both ELISA and Western blotting were significantly lower in both non-AD neurodegenerative and infectious brain diseases, compared to AD, non-specific effects of neurodegeneration or inflammation on CSF levels of cystatin C are highly unlikely (see Example 1).

In preferred embodiments, measurement of the level of transcription products of the cystatin C gene, or a variant thereof, is performed in body cells using Northern blots with probes specific for the cystatin C gene or said variant. Quantitative PCR with primer combinations to amplify cystatin C gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, said level or activity of cystatin C, or a variant thereof, is detected using an immunoassay. These assays can e.g. measure the amount of binding between cystatin C and an anti-cystatin C antibody by the use of enzymatic, chromodynamic, radioactive, or luminescent labels which are attached to either the anti-cystatin C antibody or a secondary antibody which binds the anti-cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect cystatin C or a variant thereof. It is preferred that it does not substantially interact with any other protein present in said sample. It is particularly preferred to include specific antibodies or ligands which differentiate monomers from dimers or oligomers of cystatin C. As is it shown in Example 1, the detection of the monomeric form of cystatin C may be more specific to AD than measuring dimers or oligomers. Measures might be significantly improved with monomer-specific ELISAs.

Monoclonal antibodies capable of recognizing cystatin C or a variant thereof can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2nd ed., W. H. Freeman and Company, 1992; the contents of which are incorporated herein by reference). Monoclonal antibodies useful in the methods of the invention are directed to an epitope of cystatin C or a variant thereof, such that the complex formed between the antibody and cystatin C, or between the antibody and said variant, can be recognized in detection assays. The term "antibodies" encompasses all forms of antibodies known in the art, such as polyclonal , monoclonal, chimeric, recombinatorial, single chain antibodies as well as fragments thereof which specifically bind to cystatin C, or to a variant thereof. It is particularly preferred to use specific antibodies that selectively detect cystatin C monomers, dimers or oligomers, respectively. High-affinity ligands can be prepared by using derivatives of cathepsins which are the natural substrates of cystatin C biological activity.

It is further preferred to determine e.g. the level of cystatin C and its activity on basis of an enzymatic assay. As described above, cystatin C is a cysteine protease inhibitor which binds to and regulates proteolytic activities of cathepsins. A suitable enzymatic assay can therefore be built upon the enzymatic activity of cathepsins indicating the absense or presence of different levels of cystatin C in its active, monomeric form. It is preferred to use amyloid precursor protein (APP) as a substrate. The generation of A-beta peptides as educts can be measured.

The determination of the level or activity of cystatin C, or a variant thereof, can also be performed on basis of a binding assay. Suitable binding partners include cathepsins or fragments thereof, peptides, peptidomimetics, antibodies and other chemical probes which can specifically recognize cystatin C. It is in general particularly preferred to determine cystatin C in its monomeric form.

If luminescent labels are used in any detection assay, it is preferred to use a confocal optical set-up.

In preferred emdodiments, the reference value can be that of a level, or an activity, or both said level and said activity, of at least one substance which is selected from the group consisting of cystatin C, a variant thereof, a transcription product of a cystatin C gene, and a variant thereof, in a sample, preferably a body fluid, from a subject not suffering from Alzheimer's disease. The healthy subject can be of the same weight, age, and gender as the subject who is being diagnosed or prognosed for Alzheimer's disease, or for whom an increased risk of becoming diseased with Alzheimer's disease is determined. In some cases, it might be preferred to use a reference value from the subject which is under treatment.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In another aspect, the invention features, a method of diagnosing or prognosing Alzheimer's disease in a subject, or determining whether a subject is at increased risk of becoming diseased with Alzheimer's disease. The method includes: determining the presence or absense of a mutation or polymorphism in a cystatin C gene or its non-coding regulatory elements in a sample from said subject, thereby diagnosing, or prognosing, or determining an increased risk of Alzheimer's disease in said subject. Such a mutation can e.g. be a substitution, deletion or addition of at least one base. Such mutations may result in "mis-sense" information or in "non-sense" information associated with a termination codon or a frame shift. Polymorphisms and allele variations occur more frequently in the general population but induce in principle the identical genetic alterations. Such mutations or polymorphisms may be found within the promoter region, an example of which is the Sst II polymorphic site in the promoter region of the human cystatin C gene *(CST 3).* It is preferred to determine the presence of a B allele in the cystatin C gene. The human cystatin C gene, called *CST3,* has been sequenced and its A and B alleles were also described (Balbin et al., Biol. Chem. Hoppe-Seyler, Vol. 373, 471 - 476, 1992; Abrahamson et al., Biochem. J. 268, 287 - 294, 1990; Abrahamson et al., Hum. Genet. 82, 223 - 226, 1989; the contents of these publications are incorporated herein by reference). The presence of at least one B allele in said cystatin C gene or its non-coding regulatory elements, particularly in its promoter, indicates said subject and potentially its descendants are at increased risk of developing Alzheimer's disease. In particular, homozygous *CST 3* B/B subjects are at increased risk of developing Alzheimer's disease. The data shown in Example 2 indicate an association of *CST3* B/B genotype with AD, and they suggest that inheritance of the *CST3* B/B genotype is a risk factor for AD, with estimated odds ratios of 5.34 to 10.7, but without modifying the age of onset. Thus, the *CST3* B/B genotype is a stronger risk factor for AD than inheritance of *ApoE* ε4 alleles or inheritance of the G/G or *A2M-2* alleles of the alpha-2 macroglobulin gene with odds ratios between 1.77 and 3.56.

Determining the presence or absense of a mutation or polymorphism in a cystatin C gene in a sample from said subject may comprise determining a partial nucleotide sequence of the DNA from said subject, said partial nucleotide sequence indicating the presence or absence of said mutation or polymorphism. It may further be preferred to perform a polymerase chain reaction with the DNA from said subject and subsequent restriction analysis to determine the presence or absence of said mutation or polymorphism. In a further preferred embodiment, primers depicted in SEQ ID NO. 3 and SEQ ID NO. 4 are used for amplifying the promoter region of the human cystatin C gene in order to subsequently analyze the *Sst* II polymorphic sites herein.

It is particularly preferred to determine whether such mutation or polymorphism can be found in leucin 68 codon of a human cystatin C gene which leads to a loss of *Alu* I restriction site. Such a mutation or polymorphism does not indicate diagnosis, prognosis or increased risk of Alzheimer's disease.

In another preferred embodiment, the method further includes: determining a level, or an activity, or both said level and said activity, of at least one substance which is selected from the group consisting of cystatin C, a variant thereof, a transcription product of a cystatin C gene, and a variant thereof, in a sample from said subject; and comparing said level, or said activity, or both said level and said activity, of at least one of said substances to a reference value. The association of the cystatin C genotype with the phenotype of elevated CSF levels as shown in Table 5 and Example 2 underscores the role of cystatin C in the pathophysiology of AD. Increased CSF levels of cystatin C seem to confer the increased risk for AD associated with at least one B allele in the cystatin C gene, especially with *CST3* B/B. The lowest CSF levels of cystatin C were found in healthy *CST3* A carriers.

In preferred embodiments, the sample includes tissues, cells like white blood cells or skin fibroblasts, or body fluid like e.g. cerebrospinal fluid, saliva, urine or serum plasma. For the determination of mutations or polymorphisms in the cystatine C gene, it is preferred to use DNA from body cells, in particular white blood cells.

In another aspect, the invention features, a kit for diagnosis, or prognosis, or determination of increased risk of Alzheimer's disease in a subject.

Said kit comprises:
(a) at least one reagent which is selected from the group consisting of reagents that selectively detect cystatin C, reagents that selectively detect variants thereof, reagents that selectively detect transcription products of a cystatin C gene, reagents that selectively detect variants thereof, and reagents that selectively detect mutations or polymorphisms in the cystatin C gene or its non-coding regulatory elements; and
(b) instructions for diagnosing, or prognosing, or determining whether said subject is at increased risk of developing, Alzheimer's disease by
   (i) detecting a level, or an activity, or both said level and said activity, of said cystatin C, or of said variants thereof; or of said transcription products of said cystatin C gene, or of said variants thereof, in a sample from said subject; or detecting a presence or absence of mutations or polymorphisms in said cystatin C gene or its non-coding regulatory elements in a sample from said subject; and
   (ii) diagnosing or prognosing, or determining whether said subject is at increased risk of developing, Alzheimer's disease, wherein
      a varied level, or activity, or both said level and said activity, of said cystatin C, or of said variants thereof, or of said transcription products of said cystatin C gene, or of said variants thereof, compared to a reference value representing a known health status;
      or a level, or activity, or both said level and said activity, of said cystatin C, or of said variants thereof, or of said transcription products of said cystatin C gene, or of said variants thereof, similar or equal to a reference value representing a known disease status;
      or the presence of a mutation or polymorphism in said cystatin C gene or its non-coding regulatory elements indicates a diagnosis, or prognosis, or an increased risk of Alzheimer's disease.

It is particularly preferred to determine whether such mutation or polymorphism can be found in leucin 68 codon of a human cystatin C gene which leads to a loss of *Alu* I restriction site. Such a mutation or polymorphism does not indicate diagnosis, prognosis or increased risk of Alzheimer's disease.

It is preferred that said at least one reagent and said instructions are packaged in a single container. In preferred embodiments, said reference value is that of a level, or an activity, or both said level and said activity, of at least one substance which is selected from the group consisting of cystatin C, a variant thereof, a transcription product of a cystatin C gene, and a variant thereof, in a sample from a subject not suffering from said Alzheimer's disease. The healthy subject can be of the same weight, age, and gender as the subject who is being diagnosed, or prognosed for Alzheimer's disease, or for whom an increased risk of developing Alzeheimer's disease is determined. Im some cases, it might be preferred to use a reference value of the subject which is tob be diagnosed. Said kit suitable for commercial manufacture and sale can still further include appropriate standards, positive and negative controls. It is preferred that said kit further comprises reagents to assess a function or dysfunction of said subject's kidneys.

In further preferred embodiments, the presence of at least one B allele in said cystatin C gene, in particular the presence of the B/B genotype, indicates a diagnosis, or prognosis, or an increased risk of Alzheimer's disease. In order to exclude a false positive diagnosis, it should be remarked that a mutation or polymorphism in the cystatin C gene in the codon for leucine at position 68 which abolishes an *Alu*l restriction site is not indicative for Alzheimer's disease, but for hereditary cystatin C amyloid angiopathy.

Determining the presence or absense of a mutation or polymorphism in a cystatin C gene or in its non-coding regulatory elements in a sample from said subject may comprise determining a partial nucleotide sequence of the DNA from said subject, said partial nucleotide sequence indicating the presence or absence of said mutation or polymorphism. It may further be preferred to perform a polymerase chain reaction with the DNA from said subject and subsequent restriction analysis to determine the presence or absence of said mutation. In a further preferred embodiment, primers depicted in SEQ ID NO. 3 and SEQ ID NO. 4 are used for amplifying the promoter region of the human cystatin C gene in order to subsequently analyze the *Sst* II polymorphic sites herein.

In preferred embodiments, measurement of the level of transcription products of the cystatin C gene, or a variant thereof, is performed in body cells using Northern blots with probes specific for the cystatin C gene or of said variants. Quantitative PCR with primer combinations to amplify cystatin C gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, the sample is a body fluid is e.g. cerebrospinal fluid, saliva, urine or serum plasma, or a tissue, or cells like blood cells or skin fibroblasts. For the determination of mutations or polymorphisms in the cystatin C gene, it is preferred to use DNA from body cells including fibroblasts and white blood cells. For the other analyses, it is particularly preferred to use cerebrospinal fluid. According to the present invention, an increase of the level of cystatin C in said cerebrospinal fluid from the subject relative to a reference value indicates a diagnosis, or prognosis, or increased risk of said Alzheimer's disease in said subject. As shown in Example 1, CSF levels of cystatin C, and in particular in its monomeric form (which is the one with biological activity), are significantly elevated in AD as compared to normal control subjects or to patients with non-AD neurological diseases, indicating a role of cystatin C for the early diagnosis of AD. Because CSF levels of cystatin C measured by both ELISA and Western blotting were significantly lower in both non-AD neurodegenerative and infectious brain diseases, compared to AD, non-specific effects of neurodegeneration or inflammation on CSF levels of cystatin C are highly unlikely (see Example 1).

In preferred embodiments, said level or activity of cystatin C, or a variant thereof, is detected using an immunoassay. These assays can e.g. measure the amount of binding between cystatin C and an anti-cystatin C antibody by the use of enzymatic, chromodynamic, radioactive, or luminescent labels which are attached to either the anti-cystatin C antibody or a secondary antibody which binds the anti-cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect cystatin C, or a variant thereof. It is preferred that it does not substantially interact with any other protein present in said sample. It is particularly preferred to include specific antibodies or ligands which differentiate monomers from dimers or oligomers of cystatin C. As is it shown in Example 1, the detection of the monomeric form of cystatin C may be more specific to AD than measuring dimers or oligomers. Measures might be significantly improved with monomer-specific ELISAs.

Monoclonal antibodies capable of recognizing cystatin C or a variant thereof can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2nd ed., W. H. Freeman and Company, 1992; the contents of which are incorporated herein by reference). Monoclonal antibodies useful in the methods of the invention are directed to an epitope of cystatin C or a variant thereof, such that the complex formed between the antibody and cystatin C, or between the antibody and said variant, can be recognized in detection assays. The term "antibodies" encompasses all forms of antibodies known in the art, such as polyclonal , monoclonal, chimeric, recombinatorial, single chain antibodies as well as fragments thereof which specifically bind to cystatin C, or to a variant thereof. It is particularly preferred to use specific antibodies that selectively detect cystatin C monomers, dimers or oligomers, respectively. High-affinity ligands can be prepared by using derivatives of cathepsins which are the natural substrates of cystatin C biological activity.

It is further preferred to determine the level of cystatin C and its activity on basis of an enzymatic assay. As described above, cystatin C is a cysteine protease inhibitor which binds to and regulates proteolytic activities of cathepsins. A suitable enzymatic assay can therefore be built upon the enzymatic activity of cathepsins indicating the absense or presence of different levels of cystatin C in its active, monomeric form. It is preferred to use amyloid precursor protein (APP) as a substrate. The generation of A-beta peptides as educts can be measured.

The determination of the level or activity of cystatin C can also be performed on basis of a binding assay. Suitable binding partners include cathepsins or fragments thereof, peptides, peptidomimetics, antibodies and other chemical probes which can specifically recognize cystatin C. It is in general particularly preferred to determine cystatin C in its monomeric form.

If luminescent labels are used in any detection assay, it is preferred to use a confocal optical set-up.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, said kit can also be used in monitoring a progression of Alzheimer's disease in a subject or in monitoring success or failure of a therapeutic treatment of said subject.

In another aspect, the invention features a method of treating or preventing Alzheimer's disease in a subject comprising administering to said subject in a therapeutically effective amount an agent or agents which directly or indirectly affect a biological activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof.

It is preferred that said agent or agents reduce a biological activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof.

In preferred embodiments, said agents bind or inhibit cystatin C, as e.g. cathepsin derivatives or cystatin C analoga.

In preferred embodiments, the method comprises the application of per se known methods of gene therapy and/or antisense nucleic acid technology are applied to administer said agent or agents.

In general, gene therapy includes several approaches: molecular replacement of a mutated gene, addition of a new gene resulting in the synthesis of a therapeutic protein, and modulation of endogeneous cellular gene expression by drugs. Gene-transfer techniques are described in detail (see e.g. Behr, Acc. Chem. Res. 26, 274 - 278, 1993; Mulligan, Science 260, 926 - 931, 1993; the contents of which are incorporated herein by reference) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane perturbation by chemical (solvents, detergents, polymers, enzymes) or physical means (mechanic, osmotic, thermic, electric shocks). The postnatal gene transfer into the central nervous system has been described in detail (see e.g. Wolff, Current Opinion in Neurobiology, 3, 743 - 748, 1993; the contents of which are incorporated herein by reference).

In particular, the invention features a method of treating or preventing Alzheimer's disease by means of antisense nucleic acid therapy, i.e. the down-regulation of an inappropriately expressed or defective gene by the introduction of antisense nucleic acids or derivatives thereof into certain critical cells (see e.g. Gillespie, DN & P 5(7), 389 - 395, 1992; Agrawal, Tibtech 13, 197 - 199, 1995; Crooke, Bio/Technology 10, 882 - 886, 1992; the contents of which are incorporated herein by reference). Apart from hybridization strategies, the application of ribozymes, i.e. RNA molecules that act as enzymes, destroying RNA that carries the message of disease has also been described (see e.g. Barinaga, Science, 262, 1512 - 1514, 1993; the contents of which are incorporated herein by reference). In preferred embodiments, the subject to be treated is a human and therapeutic antisense nucleic acids or derivatives thereof are directed against the human cystatin C gene *CST-3 ,* its non-coding regulatory elements, or transcription products of *CST-3.* It is particularly preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Tibtech, 10, 281 - 287, 1992; the contents of which are incorporated herein by reference). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of the target nucleic acid. Therapeutically use of intracellularly expressed antisense RNA is procedurally similar to gene therapy.

In preferred embodiments, the method comprises grafting donor cells into the central nervous system, preferably the brain, of said subject, said subject or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phospate transfection, liposomal mediated transfection, etc.

In preferred embodiments, said agent is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In preferred embodiments, the therapeutic nucleic acid or protein reduces brain amyloid formation by interacting with a cystatin C gene, its transcription products, cystatin C, or variants of the former. It is preferred that said brain amyloid is β-amyloid derived by proteolytic processing of the amyloid precursor protein (APP) of Alzheimer's disease.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In another aspect, the invention features an agent which directly or indirectly affects a biological activity, or level, or both said activity and level, of at least one substance which is selected from the group consisting of a cystatin C gene, a variant thereof, a transcription product of a cystatin C gene, a variant thereof, cystatin C and a variant thereof.

It is preferred that said agent or agents reduce(s) a biological activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof. In preferred embodiments, the agent is a therapeutic nucleic acid or protein which reduces brain amyloid formation by interacting with a cystatin C gene, its transcription products, cystatin C, or variants of the former. It is preferred that said brain amyloid is β-amyloid derived by proteolytic processing of the amyloid precursor protein (APP) of Alzheimer's disease.

In a further aspect, the invention features a medicament comprising an agent which directly or indirectly affects a biological activity, or level, or both said activity and level, of at least one substance which is selected from the group consisting of a cystatin C gene, a variant thereof, a transcription product of a cystatin C gene, a variant thereof, cystatin C and a variant thereof. It is preferred that said agent reduces a biological activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof.

In another aspect, the invention features an agent which directly or indirectly affects a biological activity, or level, or both said activity and level, of at least one substance which is selected from the group consisting of a cystatin C gene, a variant thereof, a transcription product of a cystatin C gene, a variant thereof, cystatin C and a variant thereof, for treating or preventing Alzheimer's disease. It is preferred that said agent or agents reduce a biological activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof. In preferred embodiments, the agent is a therapeutic nucleic acid or protein which reduces brain amyloid formation by interacting with a cystatin C gene, its transcription products, cystatin C, or variants of the former. It is preferred that said brain amyloid is β-amyloid derived by proteolytic processing of the amyloid precursor protein (APP) of Alzheimer's disease.

In a further aspect, the invention features the use of an agent which directly or indirectly affects a biological activity, or level, or both said activity and level, of at least one substance which is selected from the group consisting of a cystatin C gene, a variant thereof, a transcription product of a cystatin C gene, a variant thereof, cystatin C and a variant thereof, for a preparation of a medicament for treating or preventing Alzheimer's disease. It is preferred that said agent or agents reduce(s) a biological activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof. In preferred embodiments, the agent is a therapeutic nucleic acid or protein which reduces brain amyloid formation by interacting with a cystatin C gene, its transcription products, cystatin C, or variants of the former. It is preferred that said brain amyloid is β-amyloid derived by proteolytic processing of the amyloid precursor protein (APP) of Alzheimer's disease.

In another aspect, the invention features a method for identifying an agent that directly or indirectly affects an activity, or level, or both said activity and level, of at least one substance which is selected from the group consisting of a cystatin C gene, a variant thereof, a transcription product of a cystatin C gene, a variant thereof, their non-coding regulatory elements, cystatin C and a variant thereof, comprising the steps of:
(a) providing a sample containing at least one substance which is selected from the group consisting of a cystatin C gene, a variant thereof, a transcription product of a cystatin C gene, a variant thereof, their non-coding regulatory elements, cystatin C and a variant thereof;
(b) contacting said sample with at least one agent;
(c) comparing an activity, or level, or both said activity and level, of at least one of said substances before and after said contacting. It is preferred that said agent reduces an activity, or level, or both said activity and level, of at least one of said substances.

In preferred embodiments, measurement of the level of transcription products of a cystatin C gene, or a variant thereof, is performed in body cells using Northern blots with probes specific for the cystatin C gene or said variant. Quantitative PCR with primer combinations to amplify cystatin C gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, said level or activity of cystatin C, or a variant thereof, is detected using an immunoassay. These assays can e.g. measure the amount of binding between cystatin C and an anti-cystatin C antibody by the use of enzymatic, chromodynamic, radioactive, or luminescent labels which are attached to either the anti-cystatin C antibody or a secondary antibody which binds the anti-cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect cystatin C or a variant thereof. It is preferred that it does not substantially interact with any other protein present in said sample. It is particularly preferred to include specific antibodies or ligands which differentiate monomers from dimers or oligomers of cystatin C.

Monoclonal antibodies capable of recognizing cystatin C, or a variant thereof, can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2 nd., W. H. Freeman and Company, 1992; the contents of which are incorporated herein by reference). Monoclonal antibodies useful in the methods of the invention are directed to an epitope of cystatin C or a variant thereof, such that the complex formed between the antibody and cystatin C, or between the antibody and said variant, can be recognized in detection assays. The term. "antibodies" encompasses all forms of antibodies known in the art, such as polyclonal , monoclonal, chimeric, recombinatorial, single chain antibodies as well as fragments thereof which specifically bind to cystatin C or said variant. It is particularly preferred to use specific antibodies that selectively detect cystatin C monomers, dimers or oligomers, respectively. High-affinity ligands can be prepared by using derivatives of cathepsins which are the natural substrates of cystatin C biological activity.

It is further preferred to determine the level of cystatin C and its activity on basis of an. enzymatic assay. As described above, cystatin C. is a cysteine protease inhibitor which binds to and regulates proteolytic activities of cathepsins. A suitable enzymatic assay can therefore be built upon the enzymatic activity of cathepsins indicating the absense or presence of different levels of cystatin C in its active, monomeric form. It is preferred to use amyloid precursor protein (APP) as a substrate. The generation of A-beta peptides, in particular A-beta 40 and A-beta 42 peptides, as educts can be measured.

The determination of the level or activity of cystatin C, or a variant thereof, can also be performed on basis of a binding assay. Suitable binding partners include cathepsins or fragments thereof, peptides, peptidomimetics, antibodies and other chemical probes which can specifically recognize cystatin C, or a variant thereof. It is in general particularly preferred to determine cystatin C in its monomeric form.

If luminescent labels are used in any detection assay, it is preferred to use a confocal optical set-up.

Other features and advantages of the invention will be apparent from the following detailed description of the examples, and from the claims.

Table 1 shows CSF levels of cystatin C measured by ELISA or by Western blotting and densitometry in AD-patients, non-AD neurological and healthy controls.

Table 2 shows the data obtained by ELISA measurements of cystatin C in human CSF in various neurological and psychiatric diseases.

Table 3 shows the allele frequency of *CST3* B in AD and controls.

Table 4 shows the odds ratios of allelic variants for AD.

Table 5 shows the influence of the *CST3* genotype on CSF levels of cystatin C.

Table 6 shows that there is no effect of *ApoE* genotype on CSF levels of cystatin C.

Figure 1 depicts the correlation of ELISA and Western blotting assays.

Figure 2 depicts the influence of freezing and thawing of CSF samples on their cystatin C level.

Figure 3 depicts a schematic illustration of the human cystatin C gene with the exons numbered and shown as filled boxes as well as the three different alleles (A, B and C) with their respective nucleotide sequences given around the mutations in the promotor region. Nucleotide numbering for the base substitutions relates to the start site for cystatin C translation (+1 - +3 equals initiator methionine codon). The polymorphic *Sst* II and *Dde* I sites are underlined, and expected lengths of DNA fragements after respective cleavage are indicated.

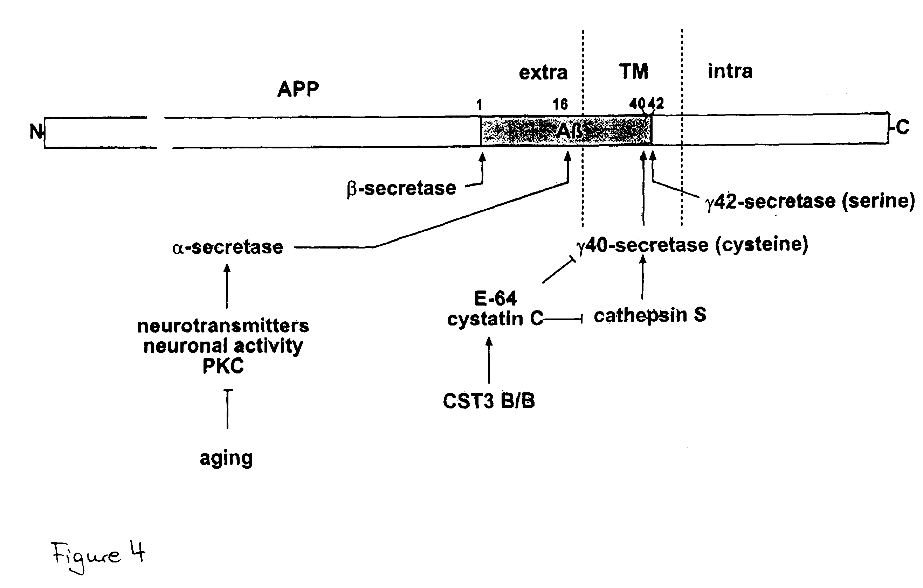

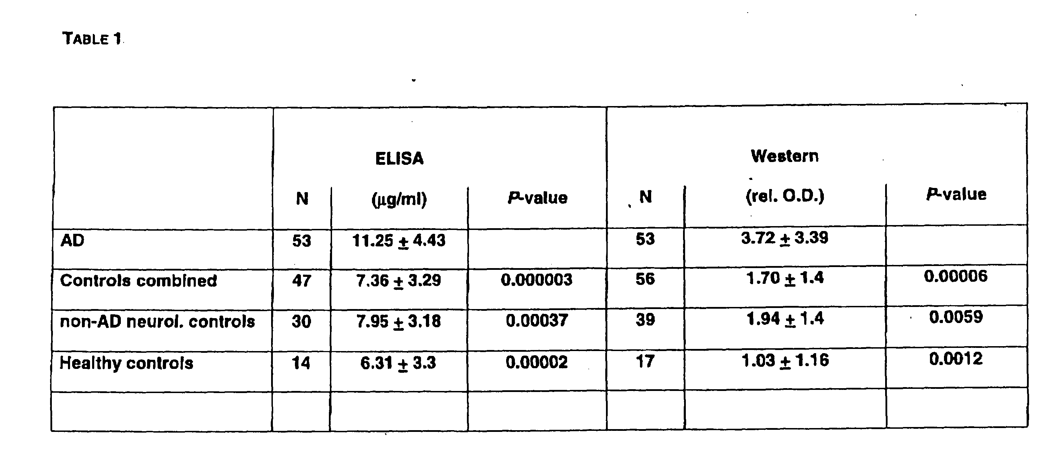

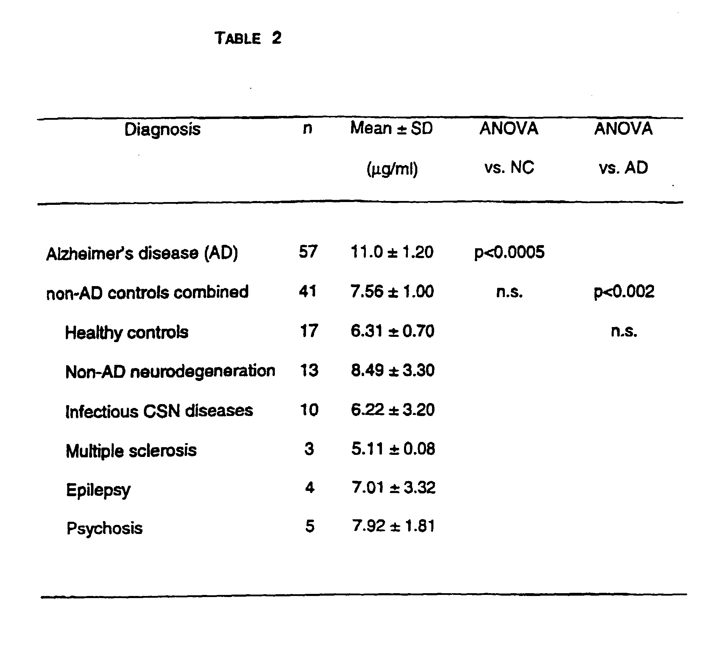

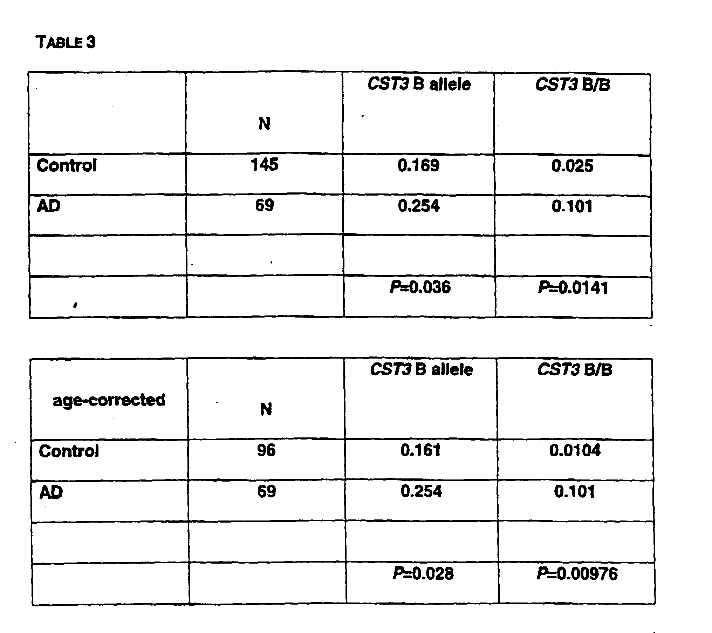

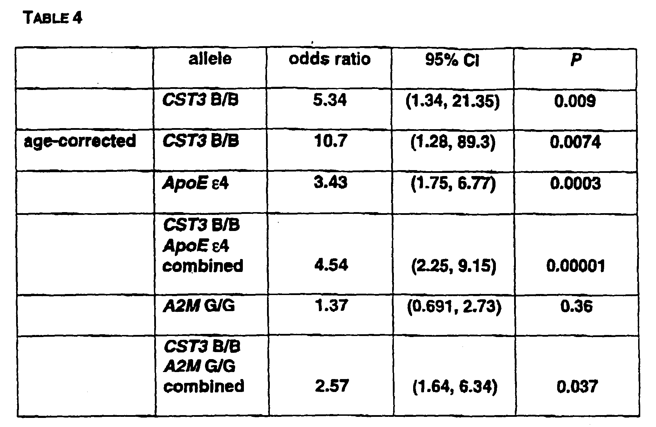

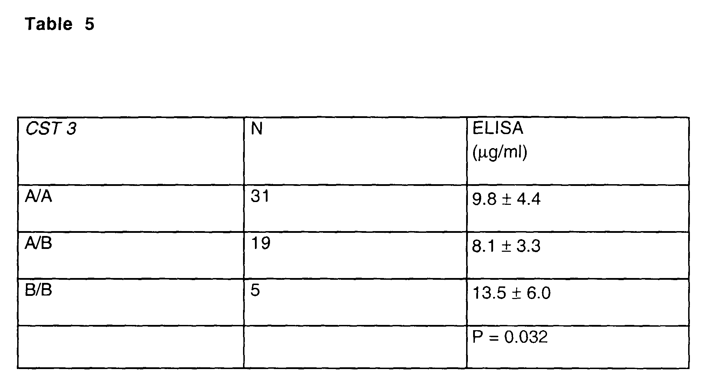

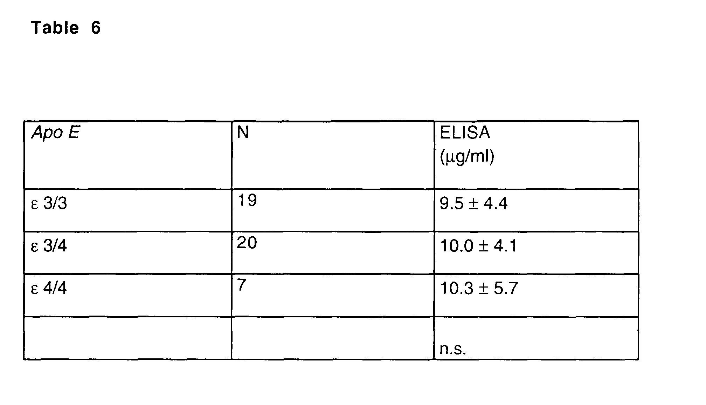

Figure 4 depicts the possible roles of cystatin C and the *Aspergillus japonicus* cysteine proteinase inhibitor E-64 in the amyloid precursor protein (APP) processing and generation of the amyloid β peptide (A_{β}).

### EXAMPLE 1

### Patient characteristics

CSF was collected by lumbar puncture from 56 patients (25 men and 31 women) with a clinical diagnosis of probable AD based on NINDS-ADRDA criteria (McKhann et al., Neurology, 34, 939 - 944, 1984). The mean age of dementia onset was 69.8 ± 8.9 years and the mean age at lumbar puncture was 73.1 ± 8.7 years. At the time of lumbar puncture, the mean score of the Mini Mental State Examination MMSE (Folstein et al., J. Psychiat. Res., 12, 189 - 198, 1975) was 18.1 ± 6.5. Among the AD patients, 18 individuals had an *ApoE* ε3/3 genotype, 27 had *ApoE* ε3/4 heterocygotes, 8 were homozygous for *ApoE* ε4/4, and 2 had an *ApoE ε2/4* genotype. One AD patient was not genotyped.

There were 38 patients (23 men and 15 women) with neurodegenerative or neurological disorders other than AD that were combined to form a non-AD neurodegenerative disease control (DDC) group. This heterogeneous group included 13 patients with Parkinson's disease, 2 with Huntington's disease, 5 with amyotrophic lateral sclerosis, 5 with epilepsy, and 4 with multiple sclerosis. Other diagnoses in this group included progressive supranuclear palsy, Binswanger's disease, cerebellar atrophy, leukodystrophy, cerebral microangiopathy, and frontal lobe degeneration, HIV-related dementias, borreliosis, alcohol-related dementias, microangiopathy and bipolar psychoses. The mean age of the patients in the DDC group was 56.5 ± 16, and the mean MMSE score was 26 ± 4.7. There were 13 DDC patients with an *ApoE* ε3/3 genotype, 4 with *ApoE* ε3/4, 3 with *ApoE ε2/3,* and 16 DDC patients were not genotyped.

CSF was also collected from 17 normal control (NC) subjects (11 men and 6 women) who were healthy at the time of lumbar puncture as determined by physical and neurological examination, and who had normal routine blood and CSF tests. Specifically, a dementing illness was excluded clinically and by cognitive testing; the mean MMSE in the normal control group was 29.0 ± 1.2. Of those with a tested *ApoE* genotype, 9 had *ApoE* ε3/3, 3 had *ApoE* ε3/4, 2 had *ApoE ε2/3.* Age was controlled for by two statistical techniques. The first was to stratify the analysis by the decade of lumbar puncture, and the other was to use age as a covatiate in an analysis of covariance. This study protocol was approved by the respective human studies and ethics committees at all participating centers.

### Lumbar puncture

At least three hours before obtaining the CSFs, patients were asked to stay in bed in order to equalize possible gradients of protein concentrations in the spinal canal. CSF was obtained by lumbar puncture with the subject in the lateral decubitus position. The initial three milliliters were used for routine cell counts and clinical chemistry, and the following six milliliters were immediately frozen at the bedside in 12 aliquots of 500 µl each. Frozen CSF samples were stored at - 80 °C until biochemical analyses.

### Enzyme-linked immunosorbent assay (ELISA)

For measuring CSF concentrations of cystatin C, a modified version of the Olafson et al. sandwich ELISA protocol was used (Olafson et al., Scand. J. Clin. Lab. Invest., 48, 573 - 582, 1988, which is hereby incorporated by reference). In brief, 50 µl of 1:500 dilutions of CSF samples or a dilution curve of purified human cystatin C (Calbiochem) were incubated in 96-well plates (Nunc Maxisorb) that were previously coated with a rabbit polyclonal anti-human cystatin C antiserum (DAKO, Germany) and blocked with 3% BSA, 0.05% Tween 20 in PBS. The biotinylated mouse monoclonal antibody HCC3 (provided by Dr. Magnus Abrahamson) against human cystatin C was used as a secondary antibody, it was detected by streptavidin conjugated with horseradish peroxidase (Amersham) and O-phenylenediamine (Sigma) as a chromagen read at 490 nm in an ELISA reader (Biorad). Diluted CSF samples were randomly distributed on the plates, assayed blinded towards diagnosis, and triplicates of each sample were measured on two separate occasions. The sensitivity of this assay was 0.5 ng/ml, it was linear over range of 1 to 200 ng/ml (r=0.998), and the coefficient of variance was less than 5% in all cases.

### Western blotting and densitometry

A Keyhole limpet hemosiderin-conjugated peptide of SEQ ID No. 1 (EGDPEAQRRVSKNSK) was used to immunize rabbits with to generate the antiserum R9672. The antiserum was affinity-purified after ammonium sulfate precipitation by binding to the above peptide conjugated to NHS-activated sepharose (HiTrap, Pharmacia). In CSF, this antiserum specifically detected the monomeric form of cystatin C that migrated on 2D gels at a pH of 8.75, with a molecular mass of 13.3 kDa, the identical protein was detected by a commercial anti-human cystatin C antibody (Amava, Switzerland). The signal generated by either antibody was preadsorbable by preincubation of the antiserum with puried cystatin C, and densitometric measurements of signal intensities generated with human CSF samples correlated significantly (r = 0.952, P < 0.003). Together, the characterization of R9672 revealed that it specifically reacted in CSF with cystatin C, despite the fact that it was raised against, and detects in brain protein extracts, the 21 kDa presenilin 1 C-terminal fragment.

For the determination of monomeric forms of cystatin C, 35 µl CSF were centrifuged and superfusate media were lyophilized by brief vacuum centriguation, resuspended in 15 µl water and 15 µl 2X reducing SDS-gel loading buffer and heated to 37 °C for 10 minutes. 25 µl of the CSF protein extracts were loaded on each lane of 16% linear SDS polyacrylamide gels, separated by electrophoresis, electrotransferred to PVDF membranes (Immobilon), and probed with affinity-purified R9672. Anti-rabbit IgG conjugated to horseradish peroxidase (Amersham), as used as secondary antibody detected by chemiluminescence with preflashed (linear) X-ray films (Kodak). At the 1:10,000 dilution used for the anti-rabbit secondary antibody there was no detectable cross-reaction with human IgG derivatives present in the CSF protein extracts. A standard was prepared from a large volume of CSF obtained from a patient with normal pressure hydrocephalus, processed identically and aliquoted in 50 µl sizes that were thawed only once before each gel run. Two standard samples were included on each gel; they were always processed identically and in parallel with the test samples. Samples were measured three times on three separate occasions. Immunoreactive bands were digitized by a flat bed scanner, and the optical densities were analyzed by the "NIH image" software package and normalized to the average signal generated by the standards on each blot. Measurements were always done in the linear range of the assay.

### Protein purification and peptide sequencing

Human CSF obtained by lumbar puncture was dialyzed against 10 mM Tris pH 7.8, loaded onto an FPLC Mono Q HR5/5 column (Pharmacia), and proteins were eluted with a stepwise gradient of 0 to 1 M NaCI in 10 mM Tris pH 7.8. Fractions were tested for immunoreactivity with R9672 by Western blotting. R9672-positive fractions eluted at 100 mM NaCI; they were separated by SDS-polyacrylamide gel electrophoresis, electroblotted onto immobilon-P^{SQ} membranes and stained with coomassie blue. The R9672-immunoreactive 13.3 kDa protein was subjected to microsequencing by Edman degradation on an automated Applied BioSystems 476A protein sequencer.

### Statistics

Statistical analysis was performed using methods know in the art. Such methods are described in detail in, for example, Snedecor G.W. and Cochran W.G., Statistical methods, 8th ed., Iowa State University Press, Ames Iowa 1989.

### Results

### Elevated CSF levels of total cystatin C in sporadic AD

CSF samples were taken from the above mentioned 56 sporadic AD patients, 38 patients with various non-AD neurological diseases (DDC), and 17 normal healthy control subjects (NC). By using ELISA, significantly higher CSF levels of cystatin C were found in AD patients than in either normal control subjects or patients with other non-AD neurological and psychiatric disorders as shown in Table 1. The non-AD neurological controls were not statistically different from the healthy controls (ELISA P = 0.177, Western P = 0.222). Samples for ELISA and Western blotting and densitometry measurements were derived from identical patients except for 9 non-AD neurological controls that were available for Western blotting only. Groups were compared by ANOVA and Scheffé-tests. Further, CSF levels of cystatin C in patients with non-AD neurological and psychiatric diseases did not differ statistically from our normal control subjects, and from normal CSF levels reported in the literature (Lofberg et al., J. Neurol. 223, 159 - 170, 1980; Grubb, New Engl. J. Med. 311, 1547 - 1549, 1984; Grubb et al., Scand. J. Clin. Lab. Invest. 45, Suppl. 177, 7 - 13, 1985) as shown in Table 2. Importantly, CSF levels of cystatin C in patients with non-AD neurodegenerative diseases, or with inflammatory CNS diseases were significantly lower than in AD patients and did not differ from normal controls. These data indicate that changes in cystatin C levels are not a non-specific response to inflammation or neurodegeneration, and argue in favor of a specific role of cystatin C in the pathophysiologie in AD. Receiver-operated characteristics analyses revealed that a cut-off value of 1.91 rel. O.D. for the monomeric form of cystatin C was associated with a specificity of 94 % and a sensitivity of 73 % for the diagnosis of Alzheimer's disease.

### Elevated CSF levels of the monomeric form of cystatin C in AD

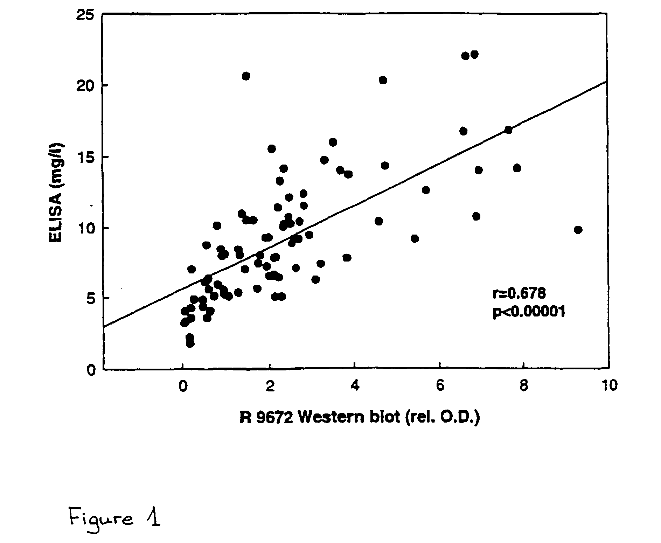

As mentioned above, cystatin C can form SDS-stable, biologically inactive dimers and oligomers. These are detected by the antibodies used for the ELISA. In order to measure CSF levels of the monomeric form of cystatin C, a quantitative Western blotting assay was used and the amount of monomeric cystatin C was determined by densitometry of the 13.3 kDa immunoreactive monomer. Data from the Western blotting assay correlated with ELISA data obtained from identical samples as shown in figure 1. Densitometric analyses revealed 3.6-fold higher concentrations in AD patients as compared to normal controls (P = 0.0012), and two-fold higher concentrations in AD patients as compared to the non-AD neurological disease group (P = 0.0059) (Table 1). As with the ELISA data, mean values for the non-AD neurological and the normal control groups did not differ. Although the results from the two assay methods were similar, the differences in group means were larger in the Western blotting assay.

The mean ages of the control group were significantly less than the mean age of the AD group. To test whether age was the determinant factor for elevated levels of the cystatin C monomer in AD, the two control groups were combined to obtain sufficient numbers of elderly patients without AD. An analysis stratifying by decade was performed, using two-way analysis of variance. This analysis also showed a significant difference between the diagnoses (P = 0.04), as did an analysis of covariance that corrected for age as a continuous covariate (P = 0.02). There was a subtle but significant correlation between age and CSF levels of cystatin C monomers for non-AD individuals (r = 0.260, P = 0.048), but not in the AD group. Together, these analyses indicate that diagnosis, rather than age, accounted for the increased CSF levels of cystatin C in AD patients.

CSF levels of the cystatin C monomer within the AD group did not vary in association with *ApoE* genotype (Table 6), nor did they correlate significantly with measures of dementia severity, age of dementia onset, duration of illness, nor gender. In addition, there was a higher variance (P < 0.01; F-test) in *ApoE* ε3 homozygous individuals with AD, caused by a subgroup of *ApoE* ε3/3 carriers with high CSF levels of cystatin C monomers. Together, these data suggest that CSF levels of cystatin C are independent of *ApoE* genotype.

Significantly more AD patients with mild to moderate stages of dementia had higher than normal (cystatin C > 2 rel. OD) CSF levels of the cystatin C monomer than severely demented patients (P < 0.01, χ² test). Comparisons of mildly demented AD patients (MMSE 18 - 30) to moderately to severely impaired patients (MMSE < 18) by χ² test also showed that more patients (P < 0.025) with mild stages of dementia had high levels of cystatin C as compared to the more severely impaired patients. This finding indicates that the metabolic abnormality leading to abnormally high CSF levels of cystatin C occurs early in the course of the disease. It raises the possibility of using CSF measurements of cystatin C in the early diagnosis of AD.

### PS1 loop antibodies crossreact with cystatin C in CSF

Even though cystatin C and PS1 do not have sequence homologies, the affinity-purified antibody R9672 strongly reacted with both the PS1 C-terminal fragment (PS1-CTF) in protein extracts prepared from human brain or transfected cells and with cystatin C derived from either human CSF or human urine. The binding of R9672 to both proteins was preadsorbable either by the PS1-derived peptide used for immunizations or by purified, denatured cystatin C. To exclude the possibility that cystatin C and a theoretical PS1-derived fragment co-migrated at 13.3 kDa on SDS gels, a two-dimensional gel electrophoresis was used to separate CSF proteins by pl in the first dimension, and by molecular mass in the second dimension. The separated proteins were transferred to Immobilon membrane filters and probed with either R9672 or with commercially available cystatin C antibodies. Both antibodies detected the same protein that migrated with 13.3 kDa at a pH of 8.75. These data matched exactly the theoretical molecular mass and pl of the 120 residue mature cystatin C after removal of the signal peptide. There were no other immunoreactive proteins detected by either antibody on this 2D Western blotting assay. In particular, there was no immunoreactive protein seen with R9672 at other pH values including 4.26, the pl of a 13.3 kDa theoretical N-terminal PS1-CTF derivative generated by presenilinase cleavage.

The R9672 Western blotting assay was used to purify the 13.3 kDa protein from human CSF by combined ion exchange chromatography and SDS-polyacrylamide electrophoresis to determine its N-terminus by automated Edman degradation sequencing. Sequence analysis unequivocally revealed SSPGXPPRLVGGPMXA (SEQ ID NO. 2), corresponding with 100 % identity to the N-terminus of cystatin C without the signal peptide. There were no additional sequences detectable in this preparation.

### EXAMPLE 2

### Patient characteristics in the genetic study

145 controls (121 cognitively normal controls and 24 cognitively normal patients with non-AD neurological diseases) and 69 AD patients were included in the genetic association study. In 55 from these, CSF samples were available for the determination of cystatin C (see description of example 1).

### Amplification and genotyping of CST3, A2M and ApoE

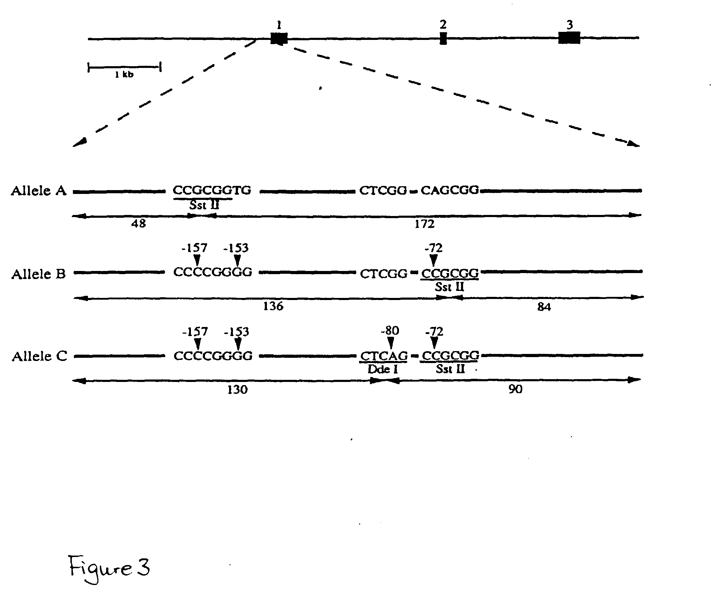

By use of polymerase chain reaction (PCR), a 318 bp segment that comprises parts of the putative promoter region of the cystatin C gene as well as the coding sequence of exon 1 (see Fig. 3), has been amplified. The primers used had sequences 5'-TGGGAGGGACGAGGCGTTCC-3' (SEQ ID NO. 3) and 5'-TCCATGGGGCCTCCCACCAG-3' (SEQ ID NO. 4. Amplification was carried out in a thermocycler under standard conditions without additives. PCR products were digested with *Sst* II or its isoschizomer *Sac* II for 1 hr at 37 °C and separated by 4 % agarose gel electrophoresis. *CST-3* B alleles derived restriction fragments are apparent in this system with 127 and 191 bp in length. *A2M* and *ApoE* were genotyped according to standard protocols.

### Results

### Genetic association of CST3 with AD

The cystatin C gene *(CST3 )* was analyzed in an exploratory case-control study of 69 patients with a clinical diagnosis of AD and 145 controls without AD. Analysis of the *Sst* II polymorphism in the 5' flanking region of *CST3 (CST3* B) revealed that it is associated with increased risk for AD. The frequency of the *CST3* B allele was 0.169 in the AD patients as compared to 0.254 in the controls (P = 0.036). The frequency of the *CST3* B/B genotype in AD was 0.101 as compared to 0.022 in controls (P = 0.0141, Fisher's exact test) as shown in Table 3. The presence of two *CST3* B alleles was associated with an odds ratio of 5.34 (95% Cl = {1.34, 21.35}, P = 0.009, Mantel-Haenszel test). By comparison, the odds ratio for *ApoE* ε4 carriers in the same data set was 3.43 (95% Cl = {1.75, 6.77}, P = 0.0003, Mantel-Haenszel test) as shown in Table 4. In an age-corrected data set of 96 controls above the age of 61 years and 70 AD patients, the frequency of *CST3* B/B was 0.0104 in the controls compared to 0.101 in AD (P = 0.00976, Fisher's exact test) as shown in Table 3. In this age-matched case-control design, the *CST3* B/B genotype was associated with an odds ratio of 10.7 (95% Cl = {1.28, 89.3}, P = 0.0074, Mantel-Haenszel test) as shown in Table 4.

The combination of either a B/B genotype and/or the presence of an *ApoE* ε4 allele resulted in an odds ratio of 4.54 (95% Cl = {2.25, 9.15}, p < 0.00001), which is higher than *ApoE* ε4 alone, but lower than *CST3* B/B alone. This difference reflects the know high number of *ApoE* ε4 positive individuals in the control group. No subjects with combined *CST3* B/B and *ApoE* ε4/4 genotypes were observed, and all control subjects with *CST3* B/B had *ApoE* ε3/3 genotypes. The *A2M* G/G genotype was not associated with AD in the present data set which is not necessarily in contrast with the reported association of *A2M* G/G because much larger sample sizes are required to detect it. The *CST3* B/B genotype was not associated with decreased ages of disease onset, analyzed either by ANOVA or by Kaplan-Meier analysis of age of onset of AD patients grouped according to *CST3* genotype.

### Elevated CSF levels of cystatin C in CST3 B/B carriers

To determine whether the *CST3* genotype influences CSF levels of cystatin C, 55'individuals from whom ELISA data of CSF levels of cystatin C were available were grouped according to *CST3* genotype *(CST3* A/A: n=31; *CST3* A/B: n = 19; *CST3* B/B: n=5). CSF levels of cystatin C in B/B carriers were significantly higher (P = 0.032, ANOVA) as compared to these in *CST3* A carriers (Table 5).

### EXAMPLE 3

All CSF samples used in Examples 1 and 2 were aliquoted and frozen on dry ice immediately upon withdrawal at the bed side, stored at - 80 °C, and thawed only immediately prior to ELISAs or Western blotting assays.

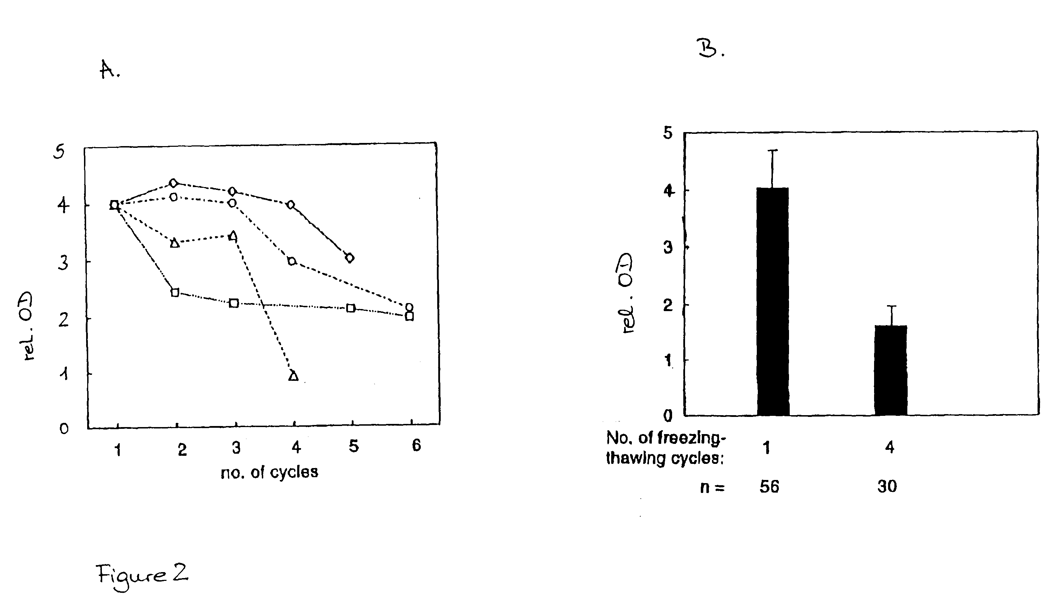

Figure 2 depicts the influence of repeated thawing and freezing CSF samples on their cystatin C content. Figure 2a shows the inter-individual variability of the sensitivity to the number of thaw-freeze cycles. In some samples, levels of the cystatin C monomer decreased 50% after two cycles, whereas in others it remained stable. After four or five freezing and thawing cycles, however, all samples consistently had significantly lower levels of the monomeric form of cystatin C. Figure 2b shows cystatin C levels after 1 and 4 freezing-thawing cycles, respectively.

CSF levels measured by ELISA were somewhat more stable towards repeated thawing and freezing, despite a clear tendency towards lower values after several cycles which might be due to the fact that the antibodies used in ELISA also detected dimers and oligomers. Consistent with this observation, low levels of cystatin C monomers were found in CSF samples derived from a source where the samples had been thawed and frozen several times prior to Western blotting. Prolonged un-interrupted freezing at - 80 °C between 1 and 114 months had no effect on CSF levels of cystatin C.

## Claims

1. A method for diagnosing or prognosing Alzheimer's disease in a subject, or determining whether a subject is at increased risk of developing Alzheimer's disease, comprising:
determining a level, or an activity, or both said level and said activity, of at least one substance which is selected from the group consisting of cystatin C, a variant thereof, a transcription product of a cystatin C gene, and a variant thereof, in a sample from said subject;
and
comparing said level, or said activity, or both said level and said activity, of at least one of said substances to a reference value representing a known disease or health status,
thereby diagnosing or prognosing said Alzheimer's disease in said subject, or determining whether said subject is at increased risk of developing Alzheimer's disease.

2. The method according to claim 1, wherein said sample is a body fluid, preferably cerebrospinal fluid.

3. The method according to claim 2, wherein an increase of said level or activity of cystatin C in said cerebrospinal fluid from said subject relative to said reference value indicates a diagnosis, or prognosis, or increased risk of said Alzheimer's disease in said subject.

4. The method according to at least one of claims 1 to 3, wherein said cystatin C is determined in its monomeric form.

5. A method of monitoring the progression of Alzheimer's disease in a subject, comprising:
determining a level, or an activity, or both said level and said activity, of at least one substance which is selected from the group consisting of cystatin C, a variant thereof, a transcription product of a cystatin C gene, and a variant thereof, in a sample from said subject;
and
comparing said level, or said activity, or both said level and said activity, of at least one of said substances to a reference value representing a known disease or health status,
thereby monitoring the progression of said Alzheimer's disease in said subject.

6. The method of claim 5, further comprising comparing a level, or an activity, or both said level and said activity, of at least one substance which is selected from the group consisting of cystatin C, a variant thereof, a transcription product of a cystatin C gene, and a variant thereof, in said sample with a level, an activity, or both said level level and said activity, of at least one of said substances in a series of samples taken from said subject over a period of time.

7. A method of evaluating a treatment for Alzheimer's disease, comprising:
determining a level, or an activity, or both said level and said activity, of at least one substance which is selected from the group consisting of cystatin C, a variant thereof, a transcription product of a cystatin C gene, and a variant thereof, in a sample obtained from a subject being treated for said Alzheimer's disease;
and
comparing said level, or said activity, or both said level and said activity, of at least one of said substances to a reference value representing a known disease or health status,
thereby evaluating said treatment for said Alzheimer's disease.

8. A method of diagnosing or prognosing Alzheimer's disease in a subject, or determining whether a subject is at increased risk of developing Alzheimer's disease comprising:
determining a presence or absence of a mutation or polymorphism in a cystatin C gene or its non-coding regulatory elements in a sample from said subject,
thereby diagnosing or prognosing Alzheimer's disease in said subject, or determining whether said subject is at increased risk of developing Alzheimer's disease.

9. The method of claim 8, wherein a presence of a mutation or polymorphism in leucin 68 codon of a human cystatin C gene leading to a loss of *Alu* I restriction site does not indicate diagnosis, or prognosis, or increased risk of Alzheimer's disease in said subject.

10. The method of claim 8 and/or 9, wherein the presence or absence of at least one B allele is determined.

11. The method of claim 10, wherein the presence of at least one B allele, in particular the presence of the B/B genotype, indicates said subject is at increased risk of developing Alzheimer's disease or indicates a diagnosis or prognosis of Alzheimer's disease.

12. A kit for diagnosis, or prognosis, or determination of increased risk of developing Alzheimer's disease in a subject, said kit comprising:
(a) at least one reagent which is selected from the group consisting of reagents that selectively detect cystatin C, reagents that selectively detect variants thereof, reagents that selectively detect transcription products of a cystatin C gene, reagents that selectively detect variants thereof, and reagents that selectively detect a mutation or polymorphism in a cystatin C gene or its non-coding regulatory elements; and
(b) instructions for diagnosing, or prognosing Alzheimer's disease, or determining increased risk of developing Alzheimer's disease by
(i) detecting a level, or an activity, or both said level and said activity, of said cystatin C, or of said variants thereof, or of said transcription products of said cystatin C gene, or of said variants thereof, in a sample from said subject; or detecting a presence or absence of a mutation or polymorphism in said cystatin C gene or its non-coding regulatory elements in a sample from said subject; and
(ii) diagnosing, or prognosing, or determining whether said subject is at increased risk of developing Alzheimer's disease,
wherein
a varied level, or activity, or both said level and said activity, of said cystatin C, or of said variants thereof, or of said transcription products of said cystatin C gene, or of said variants thereof, compared to a reference value representing a known health status;
or a level, or activity, or both said level and said activity, of said cystatin C, or of said variants thereof, or of said transcription products of said cystatin C gene, or of said variants thereof similar or equal to a reference value representing a known disease status;
or the presence of a mutation or polymorphism in said cystatin C gene or its non-coding regulatory elements indicates a diagnosis,
or prognosis, or increased risk of developing Alzheimer's disease.

13. The kit according to claim 12, wherein a presence of a mutation or polymorphism in leucin 68 codon of a human cystatin C gene leading to a loss of *Alu* I restriction site does not indicate diagnosis or prognosis of Alzheimer's disease in said subject.

14. A method of treating or preventing Alzheimer's disease in a subject comprising administering to said subject in a therapeutically effective amount an agent or agents which directly or indirectly affect an activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof.

15. The method according to claim 14, wherein said agents are cathepsin derivatives or cystatin C analoga.

16. The method according to claim 14 and/or 15 comprising grafting donor cells into the central nervous system, preferably the brain, of said subject, said subject or donor cells preferably treated so as to minimize or reduce graft rejection, wherein said donor cells are genetically modified by insertion of at least one transgene encoding said agent or agents.

17. An agent which directly or indirectly affects an activity, or level, or both said activity and level, of at least one substance which is selected from the group consisting of a cystatin C gene, a variant thereof, their non-coding regulatory elements, a transcription product of a cystatin C gene, a variant thereof, cystatin C and a variant thereof.

18. A medicament comprising an agent according to claim 17.

19. A method for identifying an agent that directly or indirectly affects an activity, or level, or both said activity and level, of at least one substance which is selected from the group consisting of a cystatin C gene, a variant thereof, a transcription product of a cystatin C gene, a variant thereof, their non-coding regulatory elements, cystatin C and a variant thereof, comprising the steps of:
(a) providing a sample containing at least one substance which is selected from the group consisting of a cystatin C gene, a variant thereof, a transcription product of a cystatin C gene, a variant thereof, their non-coding regulatory elements, cystatin C and a variant thereof;
(b) contacting said sample with at least one agent;
(c) comparing an activity, or level, or both said activity and level, of at least one of said substances before and after said contacting.

20. The method according to claim 19, wherein comparing an activity of cystatin C is performed by using amyloid precursor protein as a substrate and a generation of A-beta peptides as a read-out.
